# EUROPÄISCHE PATENTSCHRIFT

(11) **EP 2 571 887 B1**
(45) Veröffentlichungstag und Bekanntmachung des Hinweises auf die Patenterteilung: **20.08.2014**
(21) Anmeldenummer: 11719280.7
(22) Anmeldetag: 16.05.2011
(51) Int. Cl.: C07D 495/14, C07D 207/456

(54) **Verfahren zur Herstellung von Dithiin-tetracarboxy-diimiden**
Method for producing dithiin tetracarboxy-diimides
Procédé de fabrication de dithiine-tétracarboxy-diimides

(30) Priorität: 26.05.2010 US 348355 P; 21.05.2010 EP 10163519
(43) Veröffentlichungstag der Anmeldung: 27.03.2013
(73) Patentinhaber: Bayer CropScience AG, 40789 Monheim (DE)
(72) Erfinder: HIMMLER, Thomas, 51519 Odenthal (DE); KAUßMANN, Martin, 50937 Köln (DE)
(74) Vertreter: BIP Patents
(86) Internationale Anmeldenummer: PCT/EP2011/057829
(87) Internationale Veröffentlichungsnummer: WO 2011/144550

(56) Entgegenhaltungen:
- GAINA C: "Synthesis and characterization of new compounds containing 1,4-dithintetracarboxydiimide units", REVUE ROUMAINE DE CHIMIE - ROMANIAN JOURNAL OF CHEMISTRY, EDITURA ACADEMIEI ROMANE, RO, Bd. 50, Nr. 7-8, 1. Januar 2005 (2005-01-01), Seiten 601-607, XP008126359, ISSN: 0035-3930 in der Anmeldung erwähnt
- GAINA, CONSTANTIN ET AL: "Synthesis and characterization of polyamides containing 1,4-dithiin-2,3:5,6-tetrayl diimide structures", MACROMOLECULAR RAPID COMMUNICATIONS , 22(1), 25-29 CODEN: MRCOE3; ISSN: 1022-1336, 2001, XP002606528,
- GAINA C ET AL: "Polyimides containing 1,4-dithiine units and their corresponding thiophene 2,3,4,5 tetracarboxylimide units", HIGH PERFORMANCE POLYMERS, INSTITUTE OF PHYSICS PUBLISHING, BRISTOL, GB, Bd. 11, Nr. 2, 1. Juni 1999 (1999-06-01), Seiten 185-195, XP001537372, ISSN: 0954-0083
- DRABER W: "Synthese von 1.4-Dithiinen aus Derivaten des Maleinimids", CHEMISCHE BERICHTE, VERLAG CHEMIE GMBH. WEINHEIM, DE, Bd. 100, Nr. 5, 1. Januar 1967 (1967-01-01), Seiten 1559-1570, XP002585999, ISSN: 0009-2940

## Beschreibung

Die vorliegende Erfindung betrifft ein neues Verfahren zur Herstellung von Dithiin-tetracarboxy-diimiden.

Dithiin-tetracarboxy-diimide als solche sind bereits bekannt. Ebenso ist bekannt, dass diese Dithiin-tetracarboxy-diimide als Anthelminthika gegen innere Parasiten von Tieren, insbesondere Nematoden, verwendet werden können und insektizide Wirkung aufweisen (vgl. US 3,364,229). Außerdem ist bekannt, dass bestimmte Dithiintetracarboxy-diimide antibakterielle Wirkung besitzen und gegen menschliche Mykosen eine gewisse Wirkung aufweisen (vgl. Il Farmaco 2005, 60, 944-947). Auch ist bekannt, dass Dithiin-tetracarboxy-diimide als Fungizide gegen phytopathogene Pilze im Pflanzenschutz verwendbar sind (vgl. WO 2010/043319). Weiterhin ist bekannt, dass Dithiin-tetracarboxy-diimide als Pigmente in elektrophotographischen Photorezeptoren oder als Farbstoffe in Lacken und Polymeren eingesetzt werden können (vgl. JP-A 10-251265, PL-B 143804).

Dithiin-tetracarboximide der Formel (I) 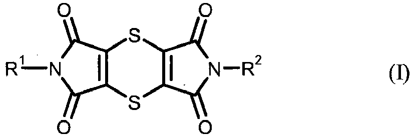 in welcher
- R¹ und R²: gleich oder verschieden sind und für Wasserstoff, für gegebenenfalls einfach oder mehrfach durch Halogen, -OR³, -COR⁴ substituiertes C₁-C₈-Akyl, gegebenenfalls einfach oder mehrfach durch Halogen, C₁-C₄-Alkyl oder C₁-C₄-Halogenalkyl substituiertes C₃-C₇-cycloalkyl, jeweils gegebenenfalls einfach oder mehrfach durch Halogen, C₁-C₄-Alkyl, C₁-C₄-Halogenalkyl, -COR⁴ oder Sulfonylamino substituiertes Aryl oder Aryl-(C₁-C₄-alkyl) stehen,
- R³: für Wasserstoff, C₁-C₄-Alkyl, C₁-C₄-Alkylcarbonyl oder für gegebenenfalls einfach oder mehrfach durch Halogen, C₁-C₄-Alkyl oder C₁-C₄-Halogenalkyl substituiertes Aryl steht,
- R⁴: für Hydroxy, C₁-C₄-Alkyl oder C₁-C₄-Alkoxy steht,
können auf verschiedene bekannte Weisen hergestellt werden.

Beispielsweise wird in einem bekannten Verfahren (vgl. Synthetic Communications 2006, 36, 3591-3597) in einer ersten Stufe Bernsteinsäureanhydrid mit einem Amin der Formel (II) gegebenenfalls in Gegenwart eines Verdünndungsmittels umgesetzt. Anschließend werden dann die so erhaltenen Bernsteinsäuremonoamide der Formel (III) mit einem großen Überschuss an Thionylchlorid in Gegenwart von Dioxan als Verdünnungsmittel bei Raumtemperatur umgesetzt, wobei in einer Abfolge zahlreicher Reaktionsschritte schließlich die Dithiin-tetracarboxy-diimide der Formel (I) erhalten werden. Die Dithiin-tetracarboxy-diimide werden wahlweise direkt aus dem Reaktionsgemisch oder nach Versetzen mit Wasser durch Filtration isoliert. Je nach Reaktionsbedingungen (Verdünnungsmittel) und Art der Reste R können unter Umständen die Dithiin-diisoimide der Formel (IV) isoliert werden, bevor man sie in die Dithiin-tetracarboxy-diimide der Formel (I) überführt:

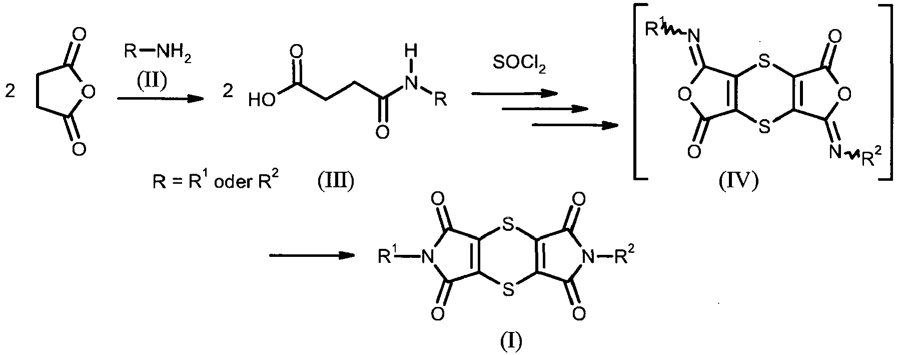

Nachteilig an diesem Verfahren sind die langen Reaktionszeit sowie das Ergebnis, dass entweder die erhaltenen Ausbeuten in der Regel etwa 30-40% der Theorie nicht überschreiten oder aber die Reinheiten der isolierten Produkte ungenügend sind. Außerdem ist bei einer wässrigen Aufarbeitung des Reaktionsgemisches nachteilig, dass dabei große Mengen Thionylchlorid vernichtet werden; die entstehenden Gase (SO₂ und HCl) müssen entsorgt werden. Ebenfalls nachteilig ist der Umstand, dass erfahrungsgemäß das Produkt nicht in einer Portion erhalten wird. Vielmehr ist es häufig so, dass nach einer ersten Produktisolierung durch Filtration aus dem Filtrat nach längerem Stehen (beispielsweise über Nacht), weiteres Produkt ausfällt, das erneut durch Filtration isoliert werden muss. Zuweilen muss dieser Vorgang nochmals durchgeführt werden. Diese Arbeitsweise ist sehr umständlich und zeitraubend.

In einem weiteren bekannten Verfahren (vgl. US 3,364,229; Chem. Ber. 1967, 100, 1559-70) wird in einer ersten Stufe Dichlormaleinsäureanhydrid der Formel (V) mit einem Amin der Formel (II) gegebenenfalls in Gegenwart eines Verdünndungsmittels umgesetzt. Anschließend werden dann die so erhaltenen Dichlor-maleinsäureimide der Formel (VI) mit einer Schwefel-freisetzenden Verbindung (beispielsweise Schwefelwasserstoff, Thioharnstoff oder Natriumthiosulfat) umgesetzt:

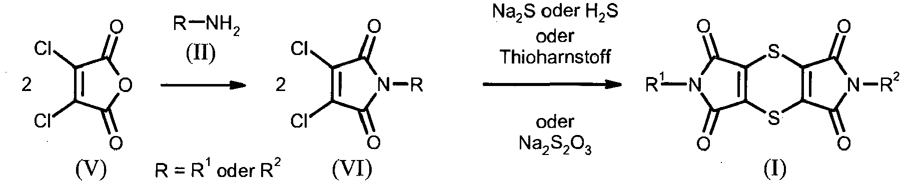

Dieses Verfahren hat die Nachteile, dass beispielsweise der Umgang mit dem hochgiftigen Schwefelwasserstoffgas technisch sehr schwierig und aufwendig ist. Bei der Verwendung von Thioharnstoff werden neben dem Zielprodukt unerwünschte Nebenprodukte erhalten, die nur sehr schwierig zu entfernen sind und die erreichbaren Ausbeuten verschlechtern. Bei der Verwendung von Natriumthiosulfat ist die beschriebene Ausbeute für einen technischen Prozess ungenügend. Entsprechendes gilt für die in der US 3,364,229 beschriebene Durchführungsform der Reaktion mit Natriumsulfid (siehe dort, Beispiel Xb).

Die Herstellung von speziellen Dithiin-tetracarboxy-diimiden der Formel (I) durch Umsetzung der entsprechenden Dichlormaleinsäureimid der Formel (VI) mit Natriumsulfid ist weiterhin auch bekannt aus Revue Roumaine de Chimie 2005, 50, 601-607. Aber auch hier sind die Ausbeuten unzureichend.

Es besteht demnach weiterhin Bedarf an einem technisch einfachen und ökonomischen Herstellverfahren für Dithiin-tetracarboxy-diimide der Formel (I).

Es wurde ein neues Verfahren zum Herstellen von Dithiin-tetracarboxy-diimiden der allgemeinen Formel (I) 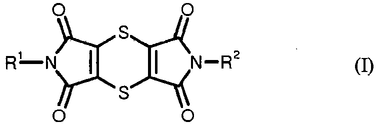 in welcher R¹ und R² die oben angegebenen Bedeutungen haben,
gefunden, dadurch gekennzeichnet, dass man
(A) Dichlormaleinsäureimide der Formel (VI) 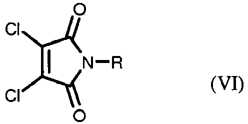 in welcher R für R¹ oder R² steht,
   mit einem anorganischen Sulfid oder Hydrogensulfid in einem Lösungsmittel oder Lösungsmittelgemisch in einem Molverhältnis zwischen 0,2 und 0,95 Mol an Sulfid oder Hydrogensulfid pro Mol Dichlormaleinsäureimid der Formel (VI) umsetzt, und
(B) das Reaktionsprodukt, welches aus einer Mischung an Verbindungen der Formel (I) und polymeren Verbindungen der allgemeinen Formel (VII) 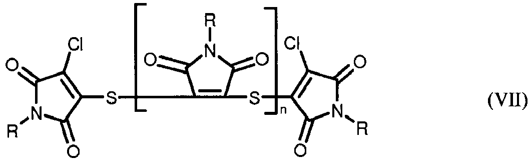 in welcher
   - R: unabhängig voneinander für R¹ oder R² steht,
   - n: für eine ganze Zahl zwischen 1 und 50 steht,
   in einem Verdünnungsmittel erwärmt.

Die bei der Durchführung des erfindungsgemäßen Verfahrens (A) als Ausgangstoffe eingesetzten Dichlormaleinsäureimide sind durch die Formel (VI) allgemein definiert. R steht für die Bedeutungen von R¹ oder R².
- R¹ und R²: sind bevorzugt gleich oder verschieden und stehen bevorzugt für Wasserstoff, für gegebenenfalls einfach oder mehrfach durch Fluor, Chlor, Brom, -OR³, -COR⁴ substituiertes C₁-C₆-Alkyl, gegebenenfalls einfach oder mehrfach durch Chlor, Methyl oder Trifluormethyl substituiertes C₃-C₇-Cycloalkyl, für jeweils gegebenenfalls einfach oder mehrfach durch Fluor, Chlor, Brom, Methyl, Trifluormethyl, -COR⁴, Sulfonylamino substituiertes Phenyl oder Phenyl-(C₁-C₄-alkyl).
- R¹ und R²: sind außerdem bevorzugt gleich oder verschieden und stehen bevorzugt für Wasserstoff, für gegebenenfalls einfach oder mehrfach durch Fluor, Chlor, Brom, -OR³ substituiertes C₁-C₆-Allcyl, gegebenenfalls einfach oder mehrfach durch Chlor, Methyl oder Trifluormethyl substituiertes C₃-C₇-C₇-Cycloalkyl.
- R¹ und R²: sind besonders bevorzugt gleich oder verschieden und stehen besonders bevorzugt für Wasserstoff, für gegebenenfalls einfach oder mehrfach durch Fluor, Chlor, Hydroxy, Methoxy, Ethoxy, Methylcarbonyloxy, Carboxyl substituiertes C₁-C₄-Alkyl, gegebenenfalls einfach oder mehrfach durch Chlor, Methyl oder Trifluormethyl substituiertes C₃-C₇-Cycloalkyl, für jeweils gegebenenfalls einfach bis dreifach durch Fluor, Chlor, Brom, Methyl, Trifluormethyl, -COR⁴, Sulfonylamino substituiertes Phenyl, Benzyl, 1-phenethyl, 2-Phenethyl oder 2-Methyl-2-phenethyl.
- R¹ und R²: sind besonders bevorzugt gleich oder verschieden und stehen besonders Bevorzugt für Wasserstoff, für gegebenenfalls einfach oder mehrfach durch Fluor, Chlor, Hydroxy, Methoxy, Ethoxy substituiertes C₁-C₄-Alkyl, gegebenenfalls einfach oder mehrfach durch Chlor, Methyl oder Trifluormethyl substituiertes C₃-C₇-Cycloakyl.
- R¹ und R²: sind ganz besonders bevorzugt gleich oder verschieden und stehen ganz besonders bevorzugt für Wasserstoff, Methyl, Ethyl, n-Propyl, Isopropyl, 2,2-Difluorethyl, 2,2,2-Trifluorethyl, jeweils gegebenenfalls durch Chlor, Methyl oder Trifluormethyl substituiertes Cyclopropyl oder Cyclohexyl.
- R¹ und R²: stehen insbesondere bevorzugt gleichzeitig für Methyl.
- R³: steht Bevorzugt für Wasserstoff, Methyl, Ethyl, Methylcarbonyl, Ethylcarbonyl oder für gegebenenfalls einfach oder mehrfach durch Fluor, Chlor, Methyl, Ethyl, n-Propyl, Isopropyl oder Trifluormethyl substituiertes Phenyl.
- R³: steht besonders bevorzugt für Wasserstoff, Methyl, Methylcarbonyl oder für Phenyl.
- R⁴: steht bevorzust für Hydroxy, Methyl, Ethyl, Methoxy oder Ethoxy.
- R⁴: steht besonders bevorzugt für Hydroxy oder Methoxy.

Besonders bevorzugt wird N-Methyl-dichlormaleinsäureimid (VI-1), R = Me, als Ausgangsstoff eingesetzt, wodurch man als Endprodukt die Verbindung (I-1) 2,6-Dimethyl-1H,5H-[1,4]dithiino[2,3-c:5,6-c']dipyrrol-1,3,5,7-(2H,6H)-tetron erhält.

Setzt man Dichlormaleinsäureimide der Formel (VI) mit einem molaren Unterschuss an Sulfid oder Hydrogensulfid um, so erhält man polymere Verbindungen der allgemeinen Formel (VII) 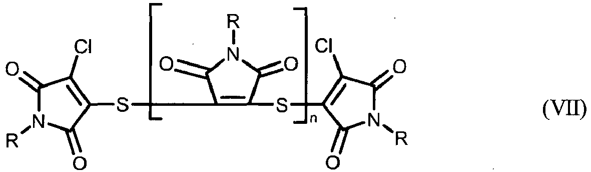 in welcher
- R: unabhängig voneinander für R¹ oder R² steht,
- n: für eine ganze Zahl zwischen 1 und 50 steht.

Polymere Verbindungen der allgemeinen Formel (VII) sind neu und ebenfalls Gegenstand der vorliegenden Erfindung. n steht bevorzugt für 1 bis 25, besonders bevorzugt für 2 bis 20, ganz besonders bevorzugt für 3 bis 15.

Als Sulfid oder Hydrogensulfid können im Prinzip alle löslichen anorganischen Sulfide oder Hydrogensulfide eingesetzt werden, wie beispielsweise Lithiumsulfid, Natriumsulfid, Natriumhydrogensulfid, Kaliumsulfid, Calciumsulfid, Calciumhydrogensulfid, Magnesiumsulfid oder Ammoniumsulfid. Bevorzugt verwendet man Natriumsulfid, Kaliumsulfid oder Ammoniumsulfid. Selbstverständlich können auch Gemische dieser Salze verwendet werden. Mit den Begriffen "Sulfid" und "Hydrogensulfid" sollen, sofern existent, auch Hydrate dieser Salze umfasst sein.

Zur Herstellung der Dithiin-tetracarboxy-diimide der Formel (I) wird das Sulfid oder Hydrogensulfid in Mengen zwischen 0,8 und 1,2 Mol pro Mol Dichlormaleinsäureimid der Formel (VI) eingesetzt. Bevorzugt sind Mengen zwischen 0,9 und 1,1 Mol, besonders bevorzugt zwischen 0,95 und 1,05 Mol, ganz besonders bevorzugt 1 Mol an Sulfid oder Hydrogensulfid pro Mol Dichlormaleinsäureimid der Formel (VI).

Zur Herstellung der polymeren Verbindungen der allgemeinen Formel (VII) wird das Sulfid oder Hydrogensulfid in Mengen zwischen 0,2 und 0,95 Mol pro Mol Dichlormaleinsäureimid der Formel (VI) eingesetzt. Bevorzugt sind Mengen zwischen 0,4 und 0,9 Mol, besonders bevorzugt zwischen 0,5 und 0,8 Mol pro Mol Dichlormaleinsäureimid der Formel (VI).

Das Sulfid oder Hydrogensulfid kann dem Reaktionsgemisch in fester Form zugesetzt werden oder als Lösung, beispielsweise in Wasser. Bevorzugt setzt man das Sulfid oder Hydrogensulfid als Lösung in Wasser zu.

Die Reaktionstemperatur des erfindungsgemäßen Verfahrens (A) kann in weiten Grenzen variiert werden und liegt zwischen -20°C und 140°C. Zur Erzielung befriedigender Raum-Zeit-Ausbeuten wird man bevorzugt bei Temperaturen zwischen -10 und 100°C arbeiten; besonders bevorzugt zwischen 0 und 50°C.

Die Reaktionszeit des erfindungsgemäßen Verfahrens (A) liegt zwischen 5 Minuten und 24 Stunden. Bevorzugt arbeitet man zwischen 10 Minuten und 12 Stunden, besonders bevorzugt zwischen 20 Minuten und 2 Stunden.

Als Lösungsmittel für das erfindungsgemäßen Verfahren (A) eignen sich Wasser, Dimethylsulfoxid, Sulfolan, Alkohole wie Methanol, Ethanol, Propanol, Isopropanol, Butanol, Tertiärbutanol, Cyclopentanol, Cyclohexanol, Ethylenglykol, Ethylenglykyol-monomethylether, Ester wie Essigsäuremethylester, Essigsäureethylester, Amide wie Formamid, N,N-Dimethylformamid; N,N-Dimethylacetamid, N-Methyl-pyrrolidon, Ether wie Tetrahydrofuran, 1,4-Dioxan, Nitrile wie Acetonitiril, Propionitril, Butyronitril, Benzonitril, Ketone wie Aceton, Methyl-ethyl-keton, Methyl-isobutylketon, Pinakolon, oder Gemische dieser Verdünnungsmittel. Bevorzugt verwendet man Wasser, Dimethylsulfoxid, Methanol, Ethanol, Propanol, Isopropanol, Butanol, Tertiärbutanol, Cyclohexanol, Ethylenglykol, Essigsäure-methylester, N,N-Dimethylformamid; N,N-Dimethylacetamid, Tetrahydrofuran, 1,4-Dioxan, Acetonitiril, Aceton, Methyl-ethyl-keton, Methyl-isobutylketon, oder Gemische dieser Verdünnungsmittel. Ganz besonders bevorzugt verwendet man Gemische aus Wasser und Methanol, Ethanol, Propanol, Isopropanol, Essigsäure-methylester, Tetrahydrofuran, 1,4-Dioxan, Acetonitiril oder Aceton.

Ein weiteres erfindungsgemäßes Verfahren (B) zur Herstellung von Dithiin-tetracarboxy-diimiden der allgemeinen Formel (I) besteht darin, ein Produkt aus der Reaktion eines Dichlormaleinsäureimids der Formel (VI) mit einem Sulfid oder Hydrogensulfid, welches vollständig oder teilweise aus Verbindungen der Formel (VII) besteht, in einem geeigneten Verdünnungsmittel zu erwärmen.

Geeignete Verdünnungsmittel zur Durchführung des erfindungsgemäßen Verfahrens (B) sind polare Lösungsmittel; beispielhaft seien hier genannt Amide wie Formamid, N,N-Dimethyl-formamid, N,N-Dimethylacetamid, N-Methyl-pyrrolidon; Alkohole wie Propanol, Isobutanol, Pentanol, Ethylenglykol; Ester wie Essigsäuremethylester, Essigsäureethylester, Butylacetat; Nitrile wie Acetonitril, Butyronitril; Ketone wie Pinakolon, Methyl-isobutylketon; Dimethylsulfoxid oder Sulfolan, oder Wasser. Diese Verdünnungsmittel können alleine oder im Gemisch mit Wasser eingesetzt werden.

Die Menge an Lösungsmittel ist nicht kritisch und kann in weiten Grenzen variiert werden.

Das erfindungsgemäße Verfahren (B) wird bei Temperaturen zwischen 0 und 200°C durchgeführt. Bevorzugt sind Temperaturen zwischen 20 und 250°C.

Die erfindungsgemäßen Verfahren werden durch folgende Beispiele veranschaulicht, ohne auf sie eingeschränkt zu sein.

### Herstellungsbeispiele

### Beispiel 1

In einem 2 Liter-Doppelmantelgefäß wurden 250 ml Methanol und 50 ml Wasser vorgelegt. Man dosierte bei 21°C innerhalb von 1 Stunde gleichzeitig erstens eine Lösung von 90 g [0,5 Mol] N-Methyl-di-chlormaleinsäureimid (VI-1) in 700 ml Methanol und zweitens eine Lösung von 66,05 g [0,5 Mol] Natriumsulfid-Trihydrat in 200 ml Wasser zu. Anschließend rührte man noch 30 Minuten bei Raumtemperatur. Man saugte den Feststoff ab, wusch ihn mit zweimal je 300 ml Wasser und dann zweimal je 300 ml Methanol und trocknete. Man erhielt auf diese Weise 64,49 g schwarzgrünen Feststoff, der laut HPLC-Analyse gegen Referenzsubstanz zu 95,1 Gewichtsprozent aus der Verbindung (I-1) besteht, was einer Ausbeute von 86,9 % der Theorie entspricht.

### Beispiel 2

Zu einer Lösung von 4,5 g [25 mmol] N-Methyl-dichlormaleinsäureimid (VI-1) in 65 ml Methanol tropfte man bei Raumtemperatur eine Lösung von 3,30 g [25 mmol] Natriumsulfid-Trihydrat in 10 ml Wasser zu. Anschließend rührte man noch 4 Stunden bei Raumtemperatur. Man saugte den Feststoff ab, wusch ihn mit 15 ml Wasser und dann 15 ml Methanol und trocknete. Man erhielt auf diese Weise 3,00 g hellgrünen Feststoff, der laut HPLC-Analyse zu 93,6 Flächenprozent aus der Verbindung (I-1) besteht, was einer Ausbeute von 79,6 % der Theorie entspricht.

### Beispiel 3

In einem 2 Liter-Doppelmantelgefäß wurden 250 ml Methanol und 50 ml Wasser vorgelegt. Man dosierte bei 10°C innerhalb von 1 Stunde gleichzeitig erstens eine Lösung von 90 g [0,5 Mol] N-Methyl-dichlormaleinsäureimid (VI-1) in 700 ml Methanol und zweitens 85,2 g 40%ige wässrige Lösung von Ammoniumsulfid [0,5 Mol], verdünnt mit 176 ml Wasser, zu. Anschließend rührte man noch 30 Minuten bei 10°C. Dann saugte man den Feststoff direkt ab, wusch ihn mit zweimal je 300 ml Wasser und dann zweimal je 300 ml Methanol und trocknete. Man erhielt auf diese Weise 64,10 g schwarzgrünen Feststoff, der laut HPLC-Analyse gegen Referenzsubstanz zu 94,4 Gewichtsprozent aus der Verbindung (I-1) besteht, was einer Ausbeute von 85,8 % der Theorie entspricht.

### Beispiel 4

Man verfuhr wie in Beispiel 3 beschrieben, jedoch unter Einsatz von 0,525 Mol Ammoniumsulfid. Man erhielt 63,56 g schwarzgrünen Feststoff, der laut HPLC-Analyse gegen Referenzsubstanz zu 96,3 Gewichtsprozent aus der Verbindung (I-1) besteht, was einer Ausbeute von 86,7 % der Theorie entspricht

### Beispiel 5

Man verfuhr wie in Beispiel 3 beschrieben, jedoch unter Einsatz von 0,55 Mol Ammoniumsulfid. Man erhielt 57,62 g schwarzgrünen Feststoff, der laut HPLC-Analyse gegen Referenzsubstanz zu 93,8 Gewichtsprozent aus der Verbindung (I-1) besteht, was einer Ausbeute von 76,8 % der Theorie entspricht.

### Beispiel 6

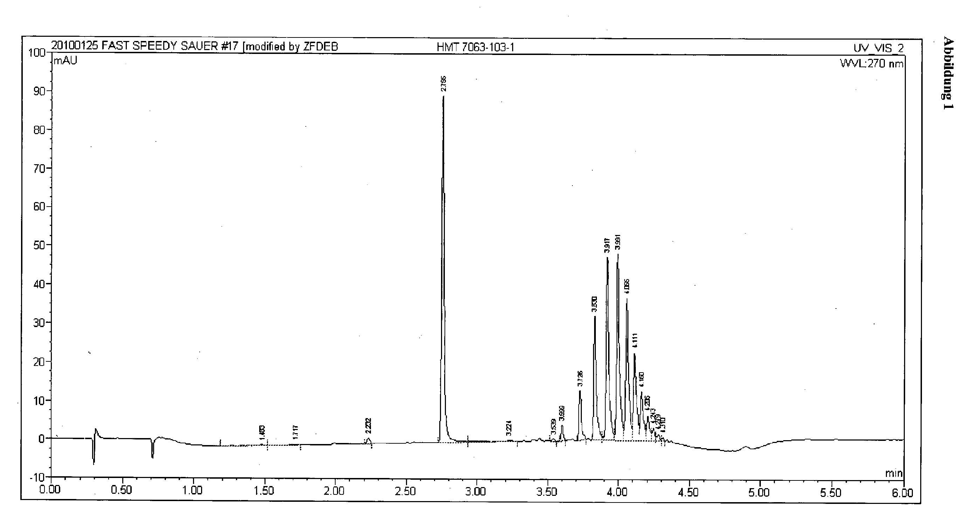

In einem 500 ml-Kolben wurde eine Lösung von 18 g [0,1 Mol] N-Methyl-dichlormaleinsäureimid (VI-1) in 260 ml Methanol vorgelegt. Bei 14-16°C wurde dann eine Lösung von 6,61 g [0,05 Mol] Natriumsulfid-Trihydrat in 40 ml zugetropft. Man ließ innerhalb von 55 Minuten auf Raumtemperatur kommen und rührte noch 60 Minuten bei Raumtemperatur. Anschließend saugte man den Feststoff ab, wusch ihn mit 60 ml Wasser und trocknete. Man erhielt so 7,05 g grünen Feststoff, der laut HPLC-Analyse (270 nm) zu 75,1 Flächenprozent aus der Verbindung (I-1) besteht. Daneben findet man in Summe etwa 22,5 Flächenprozent der Verbindungen der Formel (VII), in welcher R für Methyl steht (vgl. Abbildung 1).

Die Verbindungen der Formel (VII) (R = Methyl) wurden durch präparative HPLC isoliert.

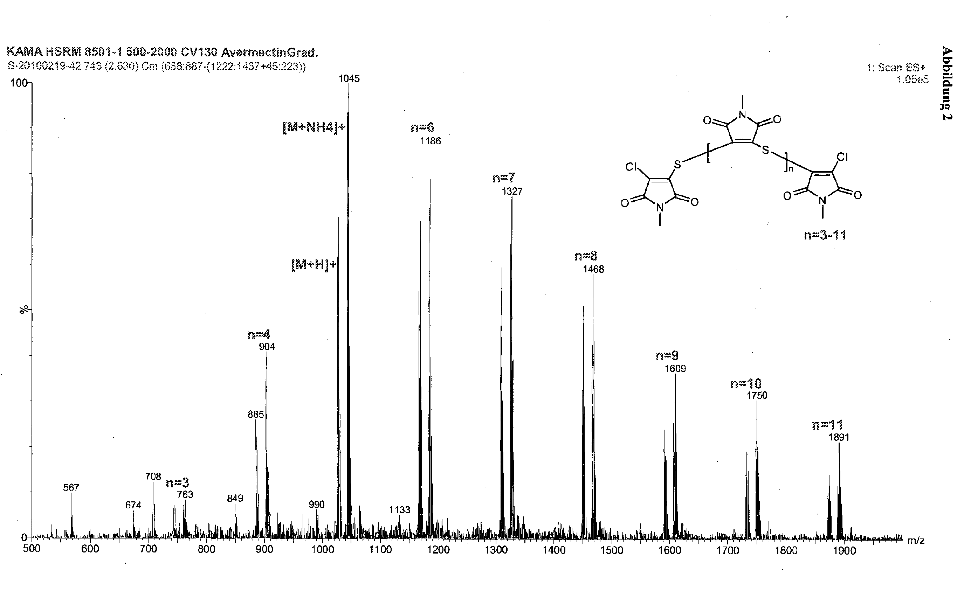

Das ESI⁺-Massenspektrum der Verbindungen der Formel (VII) mit R = Methyl wurde durch Aufsummierung der Massenspektren im Massenbereich von 500-2000 Da im Retentionszeitbereich 3.5-4.5 min erhalten. In diesem Massenbereich sind die Quasimolekülionen ([M+H]⁺ und [M+NH₄]⁺) der Oligomeren mit n = 3-11 zu erkennen (vgl. Abbildung 2).

### Beispiel 7

Man erhitzte 2 g des Produktgemisches aus Beispiel 6 in einem Gemisch aus 20 ml Ethanol und 2 ml Dimethylsulfoxid für 2 Stunden auf 77°C. Anschließend entfernte man das Lösungsmittelgemisch im Vakuum, nahm den Rückstand in 10 ml Methanol auf, saugte den Feststoff ab, wusch ihn mit 5 ml MeOH und trocknete. Man erhielt 1,77 g grünen Feststoff, der laut HPLC-Analyse (270 nm) zu 93 Flächenprozent aus der Verbindung (I-1) besteht.

### Beispiel 8

Man erhitzte 2 g eines Produktgemisches mit 64,4 Gewichtsprozent der Verbindung (I-1) in 10 ml Dimethylsulfoxid für 2 Stunden auf 100°C. Anschließend versetzte man das Reaktionsgemisch mit 30 ml Methanol, saugte den Feststoff ab, wusch ihn mit 5 ml Methanol und trocknete. Man erhielt 1,441 g schwarz-grünen Feststoff, der zu 99,4 Gewichtsprozent aus der Verbindung (I-1) besteht.

### Beispiel 9

Man erhitzte 2 g eines Produktgemisches mit 64,4 Gewichtsprozent der Verbindung (I-1) in 20 ml Butyronitril für 2 Stunden unter Rückfluss (117°C). Anschließend entfernte man das Lösungsmittel im Vakuum, versetzte den Rückstand mit 10 ml Methanol, saugte den Feststoff ab, wusch ihn mit 5 ml Methanol und trocknete. Man erhielt 1,694 g grünen Feststoff, der zu 92 Gewichtsprozent aus der Verbindung (I-1) besteht.

### Vergleichsbeispiel 1

In einem 2 Liter-Doppelmantelgefäß wurden 250 ml Methanol und 50 ml Wasser vorgelegt. Man dosierte bei 21°C innerhalb von 1 Stunde gleichzeitig erstens eine Lösung von 90 g [0,5 Mol] N-Methyl-dichlormaleinsäureimid (VI-1) in 700 ml MeOH und zweitens eine Lösung von 79,25 g [0,6 Mol] Natriumsulfid-Trihydrat in 200 ml Wasser zu. Anschließend rührte man noch 30 Minuten bei Raumtemperatur. Man saugt den Feststoff ab, wusch ihn mit zweimal je 300 ml Wasser und dann zweimal je 300 ml Methanol und trocknete. Man erhielt auf diese Weise 19,97 g hellgrünen Feststoff, der laut HPLC-Analyse zu 99,9 FI.% aus der Verbindung (I-1) besteht, was einer Ausbeute von 28,3 % der Theorie entspricht.

### Vergleichsbeispiel 2

Man verfuhr wie in Beispiel 2 beschrieben, jedoch unter Einsatz von 3,96 g [30 mmol] Natriumsulfid-Trihydrat. Nach 4 Stunden bei Raumtemperatur verblieb nach Filtration des Reaktionsgemisch kein Feststoff; d.h. die isolierte Ausbeute betrug 0 % der Theorie.

## Patentansprüche

1. Verfahren zum Herstellen von Dithiin-tetracarboxy-diiniden der allgemeinen Formel (I) 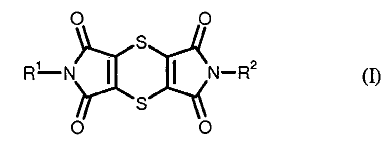 in welcher
R¹ und R² gleich oder verschieden sind und für Wasserstoff, für gegebenenfalls einfach oder mehrfach durch Halogen, -OR³, -COR⁴ substituiertes C₁-C₈-Alkyl, gegebenenfalls einfach oder mehrfach durch Halogen, C₁-C₄-Alkyl oder C₁-C₄-Halogenalkyl substituiertes C₃-C₇-Cycloalkyl, jeweils gegebenenfalls einfach oder mehrfach durch Halogen, C₁-C₄-Akyl, C₁-C₄-Ilalogenalkyl, - COR⁴ oder Sulfonylamino substituiertes Aryl oder Aryl-(C₁-C₄-alkyl) stehen,
R³ für Wasserstoff, C₁-C₄-Alkyl, C₁-C₄-Alkylcarbonyl oder für gegebenenfalls einfach oder mehrfach durch Halogen, C₁-C₄-Akyl oder C₁-C₄-Halogenalkyl substituiertes Aryl steht,
R⁴ für Hydroxy, C₁-C₄-Alkyl oder C₁-C₄-Alkoxy steht,
**dadurch gekennzeichnet, dass** man
(A) Dichlormaleinsäureimide der Formel (VI) 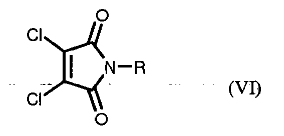 in welcher R für R¹ oder R² steht,
mit einem anorganischen Sulfid oder Hydrogensulfid in einem Lösungsmittel oder Lösungsmittelgemisch in einem Molverhältnis zwischen 0,2 und 0,95 Mol an Sulfid oder Hydrogensulfid pro Mol Dichlormaleinsäureimid der Formel (VI) umsetzt, und
(B) das Reaktionsprodukt, welches aus einer Mischung an Verbindungen der Formel (I) und polymeren Verbindungen der allgemeinen Formel (VII) 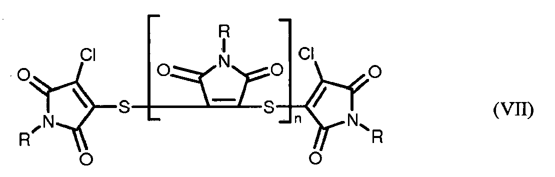 in welcher
R unabhängig voneinander für R¹ oder R² steht,
n für eine ganze Zahl zwischen 1 und 50 steht,
in einem Verdünnungsmittel erwärmt.

2. Verfahren gemäß Anspruch 1, **dadurch gekennzeichnet, dass** das Sulfid oder Hydrogensulfid aus der Gruppe bestehend aus Lithiumsulfid, Natriumsulfid, Natriumhydrogensulfid, Kaliumsulfid, Calciumsulfid, Calciumhydrogensulfid, Magnesiumsulfid oder Ammoniumsulfid oder Gemischen von diesen oder Hydraten von diesen ausgewählt ist.

3. Verfahren gemäß Anspruch 1, **dadurch gekennzeichnet, dass** das Lösungsmittel aus der Gruppe der Amide wie Formamid, N,N-Dimethyl-formamid, N,N-Dimethyl-acetamid, N-Methyl-pyrrolidon; Alkohole wie Propanol, Isobutanol, Pentanol, Ethylenglykol; Ester wie Essigsäuremethylester, Essigsäureethylester, Butylacetat; Nitrile wie Acetonitril, Butyronitril; Ketone wie Pinakolon, Methyl-isobutylketon; Dimethylsulfoxid oder Sulfolan oder Wasser oder Gemischen von diesen eingesetzt wird.

4. Polymere Verbindungen der Formel (VII) 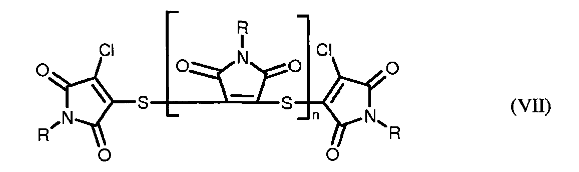 in welcher
R unabhängig voneinander für R¹ oder R² steht,
R¹ und R² gleich oder verschieden sind und für Wasserstoff, für gegebenenfalls einfach oder mehrfach durch Halogen, -OR³, -COR⁴ substituiertes C₁-C₈-Alkyl, gegebenenfalls einfach oder mehrfach durch Halogen, C₁-C₄-Akyl oder C₁-C₄-Halogenalkyl substituiertes C₃-C₇-Cycloalkyl, jeweils gegebenenfalls einfach oder mehrfach durch Halogen, C₁-C₄-Alkyl, C₁-C₄-Halogenalkyl,-COR⁴ oder Sulfonylamino substituiertes Aryl oder Aryl-(C₁-C₄-alkyl) stehen,
R³ für Wasserstoff, C₁-C₄-Akyl, C₁-C₄-Akylcarbonyl oder für gegebenenfalls einfach oder mehrfach durch Halogen, C₁-C₄-Akyl oder C₁-C₄-Halogenakyl substituiertes Aryl steht,
R⁴ für Hydroxy, C₁-C₄-Alkyl oder C₁-C₄-Alkoxy steht,
n für eine ganze Zahl zwischen 1 und 50 steht.

## Claims

1. Process for preparing dithiine-tetracarboxy-diimides of the general formula (I) 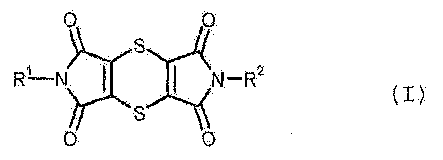 in which
R¹ and R² are identical or different and are hydrogen, or are C₁-C₈-alkyl which is optionally substituted one or more times by halogen, -OR³, and/or -COR⁴, are C₃-C₇-cycloalkyl which is optionally substituted one or more times by halogen, C₁-C₄-alkyl or C₁-C₄-haloalkyl, or are aryl or aryl- (C₁-C₄-alkyl) each of which is optionally substituted one or more times by halogen, C₁-C₄-alkyl, C₁-C₄-haloalkyl, -COR⁴ or sulphonylamino,
R³ is hydrogen, C₁-C₄-alkyl or C₁-C₄-alkylcarbonyl or is aryl which is optionally substituted one or more times by halogen, C₁-C₄-alkyl or C₁-C₄-haloalkyl,
R⁴ is hydroxyl, C₁-C₄-alkyl or C₁-C₄-alkoxy,
**characterized in that**
(A) dichloromaleimides of the formula (VI) 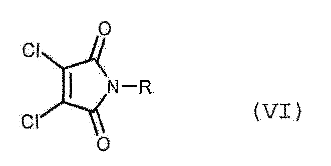 in which R is R¹ or R²
are reacted with an inorganic sulphide or hydrogen sulphide in a solvent or solvent mixture in a molar ratio between 0.2 and 0.95 mol of sulphide or hydrogen sulphide per mole of dichloromaleimide of the formula (VI), and
(B) the reaction product, which is composed of a mixture of compounds of the formula (I) and polymeric compounds of the general formula (VII) 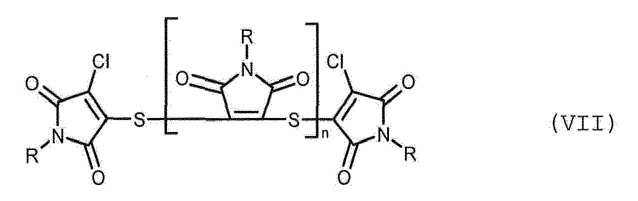 in which
R independently at each occurrence is R¹ or R², and
n is an integer between 1 and 50,
is heated in a diluent.

2. Process according to Claim 1, **characterized in that** the sulphide or hydrogen sulphide is selected from the group consisting of lithium sulphide, sodium sulphide, sodium hydrogen sulphide, potassium sulphide, calcium sulphide, calcium hydrogen sulphide, magnesium sulphide or ammonium sulphide or mixtures of these or hydrates of these.

3. Process according to Claim 1, **characterized in that** the solvent from the group of the amides such as formamide, N,N-dimethylformamide, N,N-dimethyl-acetamide, N-methylpyrrolidone; alcohols such as propanol, isobutanol, pentanol, ethylene glycol; esters such as methyl acetate, ethyl acetate, butyl acetate; nitriles such as acetonitrile, butyronitrile; ketones such as pinacolone, methyl isobutyl ketone; dimethyl sulphoxide or sulpholane or water or mixtures of these is used.

4. Polymeric compounds of the formula (VII) 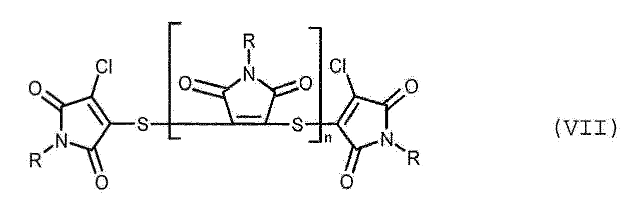 in which
R independently at each occurrence is R¹ or R²,
R¹ and R² are identical or different and are hydrogen, or are C₁-C₈-alkyl which is optionally substituted one or more times by halogen, -OR³, and/or -COR⁴, are C₃-C₇-cycloalkyl which is optionally substituted one or more times by halogen, C₁-C₄-alkyl or C₁-C₄-haloalkyl, or are aryl or aryl-(C₁-C₄-alkyl) each of which is optionally substituted one or more times by halogen, C₁-C₄-alkyl, C₁-C₄-haloalkyl, -COR⁴ or sulphonylamino,
R³ is hydrogen, C₁-C₄-alkyl or C₁-C₄-alkylcarbonyl or is aryl which is optionally substituted one or more times by halogen, C₁-C₄-alkyl or C₁-C₄-haloalkyl,
R⁴ is hydroxyl, C₁-C₄-alkyl or C₁-C₄-alkoxy,
n is an integer between 1 and 50.

## Revendications

1. Procédé de fabrication dithiine-tétracarboxy-diimides de formule générale (I) 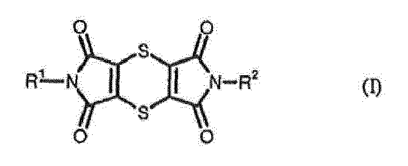 dans laquelle
R¹ et R² sont identiques ou différents et représentent hydrogène, alkyle en C₁-C₈ éventuellement substitué une ou plusieurs fois par halogène, -OR³, -COR⁴ ; cycloalkyle en C₃-C₇ éventuellement substitué une ou plusieurs fois par halogène, alkyle en C₁-C₄ ou halogénoalkyle en C₁-C₄ ; aryle ou aryl-(alkyle en C₁-C₄) chacun éventuellement substitué une ou plusieurs fois par halogène, alkyle en C₁-C₄, halogénoalkyle en C₁-C₄,-COR⁴ ou sulfonylamino,
R³ représente hydrogène, alkyle en C₁-C₄, alkylcarbonyle en C₁-C₄ ou aryle éventuellement substitué une ou plusieurs fois par halogène, alkyle en C₁-C₄ ou halogénoalkyle en C₁-C₄,
R⁴ représente hydroxy, alkyle en C₁-C₄ ou alcoxy en C₁-C₄,
**caractérisé en ce que**
(A) des imides de l'acide dichloromaléique de formule (VI) 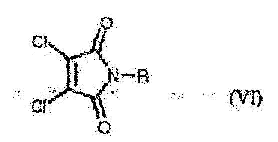 dans laquelle
R représente R¹ ou R²,
sont mis en réaction avec un sulfure ou hydrogénosulfure inorganique dans un solvant ou un mélange de solvants en un rapport en moles compris entre 0,2 et 0,95 mol de sulfure ou d'hydrogénosulfure par mol d'imide de l'acide dichloromaléique de formule (VI), et
(B) le produit de la réaction, qui est constitué d'un mélange de composés de formule (I) et de composés polymères de formule générale (VII) 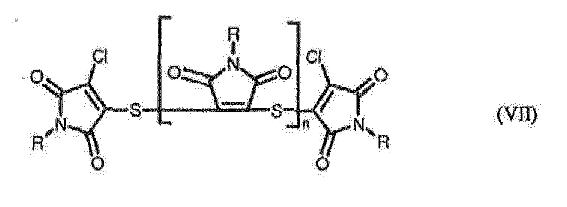 dans laquelle
les R représentent indépendamment les uns des autres R¹ ou R²,
n représente un nombre entier compris entre 1 et 50,
est chauffé dans un diluant.

2. Procédé selon la revendication 1, **caractérisé en ce que** le sulfure ou l'hydrogénosulfure est choisi dans le groupe constitué par le sulfure de lithium, le sulfure de sodium, l'hydrogénosulfure de sodium, le sulfure de potassium, le sulfure de calcium, l'hydrogénosulfure de calcium, le sulfure de magnésium ou le sulfure d'ammonium ou les mélanges de ceux-ci ou les hydrates de ceux-ci.

3. Procédé selon la revendication 1, **caractérisé en ce qu'**un solvant choisi dans le groupe constitué par les amides, tels que le formamide, le N,N-diméthyl-formamide, le N,N-diméthyl-acétamide, la N-méthyl-pyrrolidone ; les alcools, tels que le propanol, l'isobutanol, le pentanol, l'éthylène glycol ; les esters, tels que l'ester méthylique de l'acide acétique, l'ester éthylique de l'acide acétique, l'acétate de butyle ; les nitriles, tels que l'acétonitrile, le butyronitrile ; les cétones, telles que la pinacolone, la méthylisobutylcétone ; le diméthylsulfoxyde ou le sulfolane ou l'eau ou les mélanges de ceux-ci est utilisé.

4. Composés polymères de formule (VII) 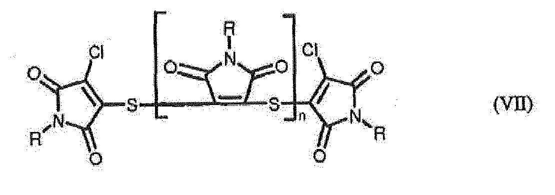 dans laquelle
les R représentent indépendamment les uns des autres R¹ ou R²,
R¹ et R² sont identiques ou différents et représentent hydrogène, alkyle en C₁-C₈ éventuellement substitué une ou plusieurs fois par halogène, -OR³, -COR⁴; cycloalkyle en C₃-C₇ éventuellement substitué une ou plusieurs fois par halogène, alkyle en C₁-C₄ ou halogénoalkyle en C₁-C₄ ; aryle ou aryl- (alkyle en C₁-C₄) chacun éventuellement substitué une ou plusieurs fois par halogène, alkyle en C₁-C₄, halogénoalkyle en C₁-C₄,-COR⁴ ou sulfonylamino,
R³ représente hydrogène, alkyle en C₁-C₄, alkylcarbonyle en C₁-C₄ ou aryle éventuellement substitué une ou plusieurs fois par halogène, alkyle en C₁-C₄ ou halogénoalkyle en C₁-C₄,
R⁴ représente hydroxy, alkyle en C₁-C₄ ou alcoxy en C₁-C₄,
n représente un nombre entier compris entre 1 et 50.
